# EUROPEAN PATENT APPLICATION

(11) **EP 0 729 755 A2**
(43) Date of publication of application: **04.09.1996**
(21) Application number: 96301306.5
(22) Date of filing: 27.02.1996
(51) Int. Cl.: A61K 31/40, A61K 31/445, A61K 31/55

(54) **2-Phenyl-3-aroylbenziothiophenen for inhibiting ovarian cancer**

(30) Priority: 28.02.1995 US 395951
(71) Applicant: ELI LILLY AND COMPANY, Indianapolis, Indiana 46285 (US)
(72) Inventor: Fuchs-Young, Robin Sharon Lee, Trafalgar, Indiana 46181 (US)
(74) Representative: Tapping, Kenneth George

(57) **Abstract**

A method of inhibiting ovarian cancer comprising administering to a human in need thereof an effective amount of a compound having the formula 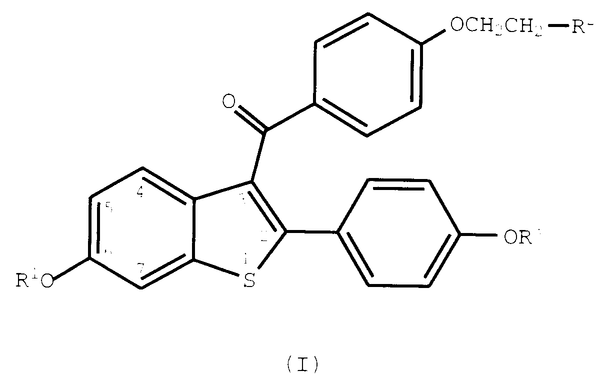
wherein R¹ and R³ are independently hydrogen, -CH₃, 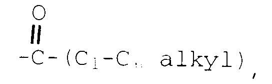
or 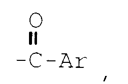
wherein Ar is optionally substituted phenyl;

R² is selected from the group consisting of pyrrolidine, hexamethyleneimino, and piperidino; or a pharmaceutically acceptable salt of solvate thereof.

## Description

Ovarian cancers account for 18% of all gynecologic neoplasms, most commonly occurring in women in their 50s. Since an ovarian neoplasm usually remains occult until it enlarges or extends enough to produce symptoms, early detection is difficult; in 70 to 80% of patients, the disease is not diagnosed until it has become extensive within or beyond the pelvis. Thus, if routine pelvic examination reveals an ovary enlarged >5 cm in diameter, close follow-up is required. In the young woman, detection of functional cysts is common; reexamination after 6 weeks will show whether spontaneous resolution has occurred. Tumor size may be the only criterion for surgery, and 1 in 4 ovarian tumors removed surgically is malignant. This ratio increases with patient age. Ovaries in postmenopausal women are small and normally not palpable. Thus, any enlargement of the ovary in a postmenopausal woman should signify a malignancy that requires prompt surgical excision. Serous cystadenocarcinoma is the most common type (found in 50% of cases) and occurs bilaterally in 30 to 50% of patients.

About 80% of malignant ovarian tumors arise from the ovarian epithelium and are classified histologically as (1) serous cystadenocarcinomas, (2) mucinous cystadenocarcinomas, (3) endometrioid tumors (similar to adenocarcinoma of the endometrium), (4) celioblastomas (Brenner tumors), (5) clear cell carcinomas, and (6) unclassified carcinomas. Tumors arising from germ cells or stroma that do not fall into this classification include granulosa-theca cell tumors, Sertoli-Leydig cell tumors, dysgerminomas, and milignant teratomas.

Ovarian cancer tends to spread by both direct extension and lymphatics to regional nodes in the pelvis and paraaortic region. With lymphatics involvement, dissemination to the abdominal and pelvic peritoneum usually occurs. With abdominal involvement, hematogenous dissemination can lead to lever and pulmonary inclusion.

An ovary may grow to considerable size before clinical symptoms appear. The earliest symptoms are vague lower abdominal discomfort and mild digestive complaints. Inappropriate endometrial bleeding is uncommon, presumably resulting from hormone secretion by the tumor. Abdominal swelling due to ovarian enlargement or accumulation of ascitic fluid, pelvic pain, anemia, and cachexia appear late in the course of disease. Additional physical signs include functional tumor effects (eg, hyperthyroidism, feminization, virilization) or, more commonly, a lobulated, fixed solid mass associated with nodular implants in the cul-de-sac. A cervical smear of the vaginal pool of pleural or peritoneal fluids may contain cells diagnostic of ovarian malignancy. X-rays may show distant metastases to lung and bone.

Although a pelvic mass and ascites usually signify a malignant ovarian tumor, a benign ovarian fibroma is rarely associated with ascites and right hydrothorax (Meigs' syndrome).

Standard therapeutic approaches are hindered by the variety of histologic types of tumors, by late discovery, and by the prevalence of widespread metastases, bilateral involvement, and extension to the uterus and contiguous structures. Not all lesions require extensive or radical therapy. In a young patient with a histologically low-grade unilateral lesion (such as a mucinous tumori, reproductive capability can be preserved by excising only the involved ovary and tube. In advanced disease, total abdominal hysterectomy and bilateral salpingo-oophorectomy with omentectomy are applied for excisable tumors, but each case involves considerable latitude. Thorough surgical staging should be performed, including biopsy of the diaphragm, parietal peritoneum, and retroperitoneal lymph nodes.

Radical surgery with node dissections and exenterations is ineffective, but radiotherapy and especially chemotherapy are becoming increasingly useful and are under continous reevaluation. When tumor involvement precludes a realistic expectation of total excision, the initial laparotomy is performed to diagnose, grade, and remove as much tumor as possible; chemotherapy or abdominal radiotherapy follows.

Widespread disease and stage IV tumors are treated with chemotherapy after adequate surgical excision. Most oncologists agree that in patients with advanced stages III and IV tumors, combination therapy with ≥ 2 cytotoxic agents is preferable to single-agent treatment.

For patients with common epithelial tumors, the overall 5-yr survival rate without evidence of recurrence is 15 to 45%. Prognosis for the rare germ cell and stromal tumors varies considerably, depending upon the stage at diagnosis. For recurrent disease, the factor most strongly correlated with survival is the time betwen diagnosis and start of multiagent therapy. With agressive tumor-reductive surgery and combination chemotherapy, the long-term survival rate for all ovarian cancer patients is improving (67% with stage III tumors in one series). However, the death rate from ovarian cancer surpasses that from cervical and corpus cancer combined and ranks 5th in cancer fatalities in women.

This invention provides methods for inhibiting ovarian cancer comprising administering to a human in need thereof an effective amount of a compound of formula I 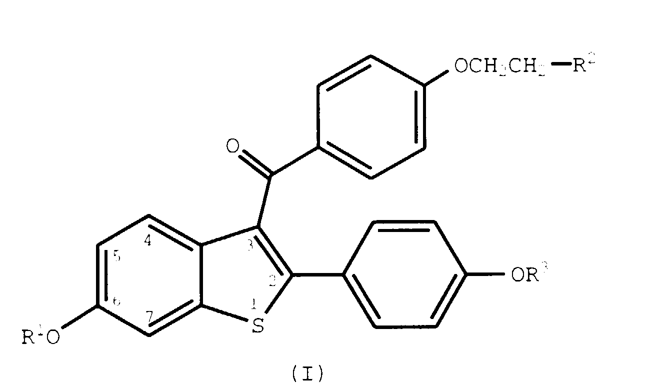 wherein R¹ and R³ are independently hydrogen, -CH₃, 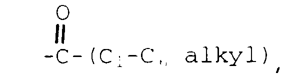 or 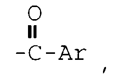 wherein Ar is optionally substituted phenyl;
R² is selected from the group consisting of pyrrolidino, hexamethyleneimino, and piperidino; and pharmaceutically acceptable salts and solvates thereof.

The current invention concerns the discovery that a select group of 2-phenyl-3-aroylbenzothiophenes (benzothiophenes), those of formula I, are useful for inhibiting ovarian cancer.

The methods of use provided by this invention are practiced by administering to a human in need thereof a dose of a compound of formula I or a pharmaceutically acceptable salt or solvate thereof, that is effective to inhibit ovarian cancer.

The term "inhibit" includes its generally accepted meaning which includes prohibiting, preventing, restraining, and slowing, stopping or reversing. As such, the present method includes both medical therapeutic and/or prophylactic administration, as appropriate.

Raloxifene, a compound of this invention wherein it is the hydrochloride salt of a compound of formula 1, R¹ and R³ are hydrogen and R² is 1-piperidinyl, is a nuclear regulatory molecule. Raloxifene has been shown to bind to the estrogen receptor and was originally thought to be a molecule whose function and pharmacology was that of an anti-estrogen in that it blocked the ability of estrogen to activate uterine tissue and estrogen dependent breast cancers. Indeed, raloxifene does block the action of estrogen in some cells; however in other cell types, raloxifene activates the same genes as estrogen does and displays the same pharmacology, e.g., osteoporosis, hyperlipidemia. As a result, raloxifene has been referred to as an anti-estrogen with mixed agonist-antagonist properties. The unique profile which raloxifene displays and differs from that of estrogen is now thought to be due to the unique activation and/or suppression of various gene functions by the raloxifene-estrogen receptor complex as opposed to the activation and/or suppression of genes by the estrogen-estrogen receptor complex. Therefore, although raloxifene and estrogen utilize and compete for the same receptor, the pharmacological outcome from gene regulation of the two is not easily predicted and is unique to each.

Generally, the compound is formulated with common excipients, diluents or carriers, and compressed into tablets, or formulated as elixirs or solutions for convenient oral administration, or administered by the intramuscular or intravenous routes. The compounds can be administered transdermally, and may be formulated as sustained release dosage forms and the like.

The compounds used in the methods of the current invention can be made according to established procedures, such as those detailed in U.S. Patent Nos. 4,133,814, 4,418,068, and 4,380,635 all of which are incorporated by reference herein. In general, the process starts with a benzo[b]thiophene having a 6-hydroxyl group and a 2-(4-hydroxyphenyl) group. The starting compound is protected, acylated, and deprotected to form the formula I compounds. Examples of the preparation of such compounds are provided in the U.S. patents discussed above. Optionally substituted phenyl includes phenyl and phenyl substituted once or twice with C₁-C₆ alkyl, C₁-C₄ alkoxy, hydroxy, nitro, chloro, fluoro, or tri(chloro or fluoro)methyl.

The compounds used in the methods of this invention form pharmaceutically acceptable acid and base addition salts with a wide variety of organic and inorganic acids and bases and include the physiologically acceptable salts which are often used in pharmaceutical chemistry. Such salts are also part of this invention. Typical inorganic acids used to form such salts include hydrochloric, hydrobromic, hydroiodic, nitric, sulfuric, phosphoric, hypophosphoric and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, teraphthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzenesulfonate, p-bromophenylsulfonate, chlorobenzenesulfonate, ethanesulfonate, 2-hydroxyethanesulfonate, methanesulfonate, naphthalene-1-sulfonate, naphthalene-2-sulfonate, p-toluenesulfonate, xylenesulfonate, tartarate, and the like. A preferred salt is the hydrochloride salt.

The pharmaceutically acceptable acid addition salts are typically formed by reacting a compound of formula I with an equimolar or excess amount of acid. The reactants are generally combined in a mutual solvent such as diethyl ether or benzene. The salt normally precipitates out of solution within about one hour to 10 days and can be isolated by filtration or the solvent can be stripped off by conventional means.

Bases commonly used for formation of salts include ammonium hydroxide and alkali and alkaline earth metal hydroxides, carbonates, as well as aliphatic and primary, secondary and tertiary amines, aliphatic diamines. Bases especially useful in the preparation of addition salts include ammonium hydroxide, potassium carbonate, methylamine, diethylamine, ethylene diamine and cyclohexylamine.

The pharmaceutically acceptable salts generally have enhanced solubility characteristics compared to the compound from which they are derived, and thus are often more amenable to formulation as liquids or emulsions.

Pharmaceutical formulations can be prepared by procedures known in the art. For example, the compounds can be formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

The compounds can also be formulated as elixirs or solutions for convenient oral administration or as solutions appropriate for parenteral administration, for instance by intramuscular, subcutaneous or intravenous routes. Additionally, the compounds are well suited to formulation as sustained release dosage forms and the like. The formulations can be so constituted that they release the active ingredient only or preferably in a particular part of the intestinal tract, possibly over a period of time. The coatings, envelopes, and protective matrices may be made, for example, from polymeric substances or waxes.

The regimen and particular dosage of a compound of formula I required to inhibit ovarian cancer according to this invention will depend upon the severity of the condition, the route of administration, and related factors that will be decided by the attending physician. Generally, accepted and effective daily doses will be from about 0.1 to about 1000 mg/day, and more typically from about 50 to about 200 mg/day. Such dosages will be administered to a subject in need thereof from once to about three times each day, or more often as needed, and for a time sufficient to effectively inhibit ovarian cancer.

It is usually preferred to administer a compound of formula I in the form of an acid addition salt, as is customary in the administration of pharmaceuticals bearing a basic group, such as the piperidino ring. It is also advantageous to administer such a compound by the oral route. For such purposes the following oral dosage forms are available.

### Formulations

In the formulations which follow, "Active ingredient" means a compound of formula I.

### Formulation 1: Gelatin Capsules

Hard gelatin capsules are prepared using the following:

| Ingredient | Quantity (mg/capsule) |
|---|---|
| Active ingredient | 0.1 - 1000 |
| Starch, NF | 0 - 650 |
| Starch flowable powder | 0 - 650 |
| Silicone fluid 350 centistokes | 0 - 15 |

The ingredients are blended, passed through a No. 45 mesh U.S. sieve, and filled into hard gelatin capsules.

Examples of specific capsule formulations of raloxifene that have been made include those shown below:

### Formulation 2: Raloxifene capsule

| Ingredient | Quantity (mg/capsule) |
|---|---|
| Raloxifene | 1 |
| Starch, NF | 112 |
| Starch flowable powder | 225.3 |
| Silicone fluid 350 centistokes | 1.7 |

### Formulation 3: Raloxifene capsule

| Ingredient | Quantity (mg/capsule) |
|---|---|
| Raloxifene | 5 |
| Starch, NF | 108 |
| Starch flowable powder | 225.3 |
| Silicone fluid 350 centistokes | 1.7 |

### Formulation 4: Raloxifene capsule

| Ingredient | Quantity (mg/capsule) |
|---|---|
| Raloxifene | 10 |
| Starch, NF | 103 |
| Starch flowable powder | 225.3 |
| Silicone fluid 350 centistokes | 1.7 |

### Formulation 5: Raloxifene capsule

| Ingredient | Quantity (mg/capsule) |
|---|---|
| Raloxifene | 50 |
| Starch, NF | 150 |
| Starch flowable powder | 397 |
| Silicone fluid 350 centistokes | 3.0 |

The specific formulations above may be changed in compliance with the reasonable variations provided.

A tablet formulation is prepared using the ingredients below:

### Formulation 6: Tablets

| Ingredient | Quantity (mg/capsule) |
|---|---|
| Active ingredient | 0.1 - 1000 |
| Cellulose, microcrystalline | 0 - 650 |
| Silicon dioxide, fumed | 0 - 650 |
| Stearate acid | 0- 15 |

The components are blended and compressed to form tablets.

Alternatively, tablets each containing 0.1 - 1000 mg of active ingredient are made up as follows:

### Formulation 7: Tablets

| Ingredient | Quantity (mg/capsule) |
|---|---|
| Active ingredien | 0.1 - 1000 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60° C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.1 - 1000 mg of medicament per 5 mL dose are made as follows:

### Formulation 8: Suspensions

| Ingredient | Quantity (mg/5 ml) |
|---|---|
| Active ingredient | 0.1 - 1000 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The medicament is passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.

### Cell Culture Assay

Continuous cell lines derived from ovarian neoplasms are maintained in culture. Raloxifene is assayed for its ability to inhibit estrogen induced effect including proliferation or up or down regulation of estrogen regulated genes. The effects of estrogen can be augmented or altered by coincident additions of appropriate cytokines, growth factors or peptide hormones. Inhibition of estrogen induced effects is evidence of activity in these cells.

### Assay 1

Between 3 and 20 women diagnosed as having ovarian cancer are used in this Assay. The stage of the cancer is noted, and the women are given raloxifene in an amount of between 50 to 600 mg/day. The period of administration is 3 months.

The women are observed during the period of administration and 3 months after discontinuance of the compound for the effects on the ovarian cancer.

### Assay 2

The same procedure is used as in Assay 1, except the period of administration is 6 months.

### Assay 3

The same procedure is used as in Assay 1, except the period of administration is 1 year.

Activity in at least one of the above tests indicates the compounds of the invention are of potential use in the treatment of ovarian cancer.

## Claims

1. The use of a compound having the formula 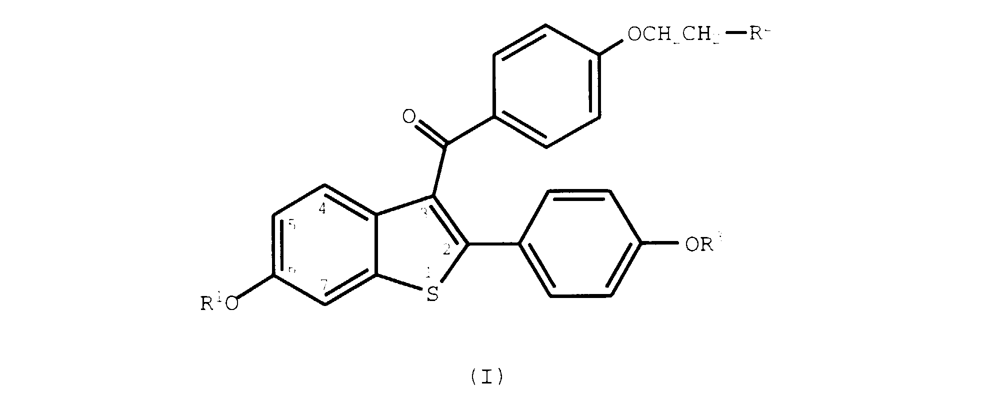 wherein R¹ and R³ are independently hydrogen, -CH₃, 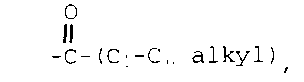 or 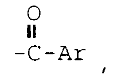 wherein Ar is optionally substituted phenyl;
R² is selected from the group consisting of pyrrolidine, hexamethyleneimino, and piperidino; or a pharmaceutically acceptable salt of solvate thereof, in the preparation of a medicament useful for inhibiting ovarian cancer.

2. The use of Claim 1 wherein said compound is the hydrochloride salt thereof.

3. The use of Claim 1 wherein said compound is 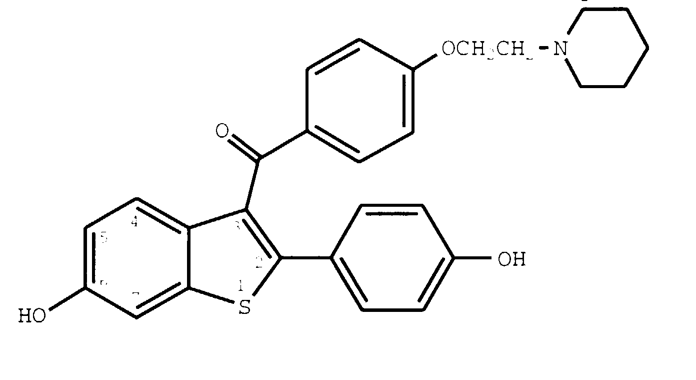 or its hydrochloride salt.
